# FASCICULE DE BREVET EUROPEEN

(11) **EP 1 706 705 B1**
(45) Date de publication et mention de la délivrance du brevet: **18.11.2009**
(21) Numéro de dépôt: 05717461.7
(22) Date de dépôt: 21.01.2005
(51) Int. Cl.: A61B 3/12, G01B 9/02

(54) **TOMOGRAPHIE A HAUTE RESOLUTION LATERALE ET AXIALE DE LA RETINE**
SEITLICH UND AXIAL HOCHAUFGELÖSTE NETZHAUTTOMOGRAFIE
HIGH RESOLUTION LATERAL AND AXIAL TOMOGRAPHY OF THE RETINA

(30) Priorité: 22.01.2004 FR 0400582
(43) Date de publication de la demande: 04.10.2006
(73) Titulaire: CENTRE NATIONAL DE LA RECHERCHE SCIENTIFIQUE (CNRS), 75794 Paris Cedex 16 (FR); Observatoire de Paris, 75014 Paris (FR); Mauna Kea Technologies, 75010 Paris (FR)
(72) Inventeur: LACOMBE, François, F-92370 Chaville (FR); LAFAILLE, David, 76600 LE HAVRE (FR); GLANC, Marie, F-92190 Meudon (FR); GENDRON, Eric, F-92190 Meudon (FR)
(74) Mandataire: Pontet, Bernard
(86) Numéro de dépôt international: PCT/FR2005/000132
(87) Numéro de publication internationale: WO 2005/080911

(56) Documents cités:
- EP-B- 1 164 921

## Description

La présente invention concerne un système de tomographie in vivo à haute résolution latérale et axiale de la rétine humaine. Elle vise également un procédé mis en oeuvre dans ce système.

WO03/105678 décrit un système et une méthode de tomographie oculaire comprenant un interféromètre de Michelson adapté pour OCT avec balayage en Z; des moyens d'optique adaptative tels qu'une source lumineuse, un miroir déformable et des moyens d'analyse de surface d'onde prévus pour corriger des fronts d'onde; un détecteur pour produire une image interférométrique.

Pour réaliser une tomographie à haute résolution axiale d'un tissu biologique, il existe déjà la technique bien connue de tomographie par interférométrie à faible longueur de cohérence (OCT, pour Optical Coherence Tomography). On peut citer par exemple les travaux de M.E. Brezinski et J.G. Fujimoto, notamment dans l'article « Optical Coherence Tomography in Non Transparent Tissue », IEEE J Sel Topics in Quant Elect, 5 :1185, 1999. Cette technique s'appuie sur un dispositif de type interféromètre de Michelson, qui consiste à faire interférer entre eux deux faisceaux lumineux issus d'une même source, dont l'un se réfléchit sur un miroir de référence et l'autre sur un échantillon à sonder. L'éclairage du dispositif par une source à faible longueur de cohérence permet de n'obtenir des interférences qu'avec la lumière renvoyée par une épaisseur de l'échantillon réalisant l'égalité des chemins optiques dans les deux bras, à mieux que la moitié de la longueur de cohérence temporelle dans le milieu.

Lorsque l'échantillon à sonder est placé dans un milieu aberrant, ou après une optique aberrante, telle que celle de l'oeil par exemple, les faisceaux aller et retour vers et depuis l'échantillon sont affectés par des aberrations géométriques. Il en résulte deux conséquences importantes :
chaque point de la source du dispositif, étendue dans le cas d'un système plein champ, voit son image dans le volume de l'échantillon dégradée par les aberrations géométriques : la zone éclairée est plus grande, voir même multiple si des tavelures ou « speckles » éclatent l'image. Cet effet d'étalement dans l'échantillon se traduit par un mélange spatial de l'information en retour, et donc par une perte de résolution spatiale. Il se traduit également par une baisse d'éclairement, donc par une baisse de sensibilité.

Sur le trajet retour, chaque point de l'échantillon donne lieu à un front d'onde, qui de nouveau altéré par les aberrations géométriques, ne peut interférer que partiellement avec le faisceau de retour du bras de référence, par manque de cohérence mutuelle des fronts d'onde. Le contraste attendu des franges d'interférence s'en trouve diminué par un facteur e^{-σ2} où σ² est la variance spatiale de phase du front d'onde perturbé. Ce phénomène est bien connu des astronomes interférométristes, qui ne peuvent concevoir d'interféromètres à plusieurs télescopes que lorsque les pupilles de ces derniers sont cohérentes, soit naturellement à grande longueur d'onde, soit après restauration par un système d'optique adaptative. Dans le cas d'un système OCT, la perte de contraste se traduit directement par une perte de sensibilité.

Ainsi, un point source en entrée n'est plus conjugué à un point unique de l'échantillon, même à la diffraction près, et a fortiori encore moins avec le détecteur placé en sortie, alors que c'est toujours le cas pour le faisceau circulant sur le bras de référence.

A ces limitations dues aux aberrations géométriques, vient s'ajouter une difficulté intrinsèque à la technique OCT : à un changement de distance d'observation par déplacement d'un miroir de référence, doit correspondre un changement de distance de focalisation (sans variation additionnelle de la différence de marche) dans l'échantillon, sans quoi de nouveau il y aura perte de contraste.

Il en résulte qu'un système de tomographie utilisé dans un milieu aberrant voit sa résolution spatiale et sa sensibilité simultanément diminuées du fait des aberrations géométriques et des changements de focalisation.

Un système de tomographie OCT peut, grâce au couplage avec un procédé d'optique adaptative (OA), voir sa sensibilité et sa résolution spatiale améliorées lorsqu'il est utilisé dans des milieux ou avec des optiques engendrant des aberrations géométriques importantes, à fortiori lorsque ces aberrations varient dans le temps. L'optique adaptative est une technique de restauration de fronts d'onde, qui s'appuie sur une mesure des perturbations du front d'onde et sur une correction en boucle fermée de ce front d'onde via un système correcteur. Il existe différentes manières de mesurer un front d'onde, donc différents types d'analyseurs. L'analyseur de type Shack-Hartmann est le plus utilisé, comme l'illustrent les documents US6,299,311 et US5,777,719. Appliquée à l'oeil, la mesure du front d'onde est réalisée sur le faisceau de retour d'un point lumineux imagé sur la rétine. Il existe également différents types de systèmes correcteurs, les miroirs déformables étant les plus courants.

Le couplage OCT+OA a déjà été envisagé comme solution d'imagerie tridimensionnelle 3D pour des milieux biologiques. Toutefois, les niveaux de sensibilité actuellement obtenus avec des systèmes implémentant un tel couplage sont nettement insuffisants pour envisager un système de tomographie in vivo pour l'examen d'une rétine humaine, pour lequel les conditions de mesure sont très difficiles compte tenu des mouvements oculaires.

Le but de l'invention est de proposer un système de tomographie à haute résolution axiale et latérale de la rétine humaine, implémentant un couplage OCT+OA et permettant une tomographie in vivo.

Cet objectif est atteint avec un système de tomographie in vivo à haute résolution axiale et latérale de la rétine humaine, comprenant :
- un interféromètre de Michelson, réalisant un montage de tomographie par interférence à faible longueur de cohérence (OCT) plein champ avec un balayage en Z,
- une source lumineuse d'entrée disposée en un bras d'entrée de l'interféromètre,
- des moyens d'optique adaptative, disposés entre l'interféromètre et un oeil à examiner, prévus pour corriger des fronts d'onde en provenance de l'oeil et à destination de l'oeil, comprenant une source de référence, un miroir déformable et des moyens d'analyse de surface d'onde,
- des moyens de détection, disposés en un bras d'imagerie de l'interféromètre, prévus pour produire une image à partir d'une mesure interférométrique selon le principe de l'OCT, et
- des moyens pour régler la focalisation des moyens d'analyse de surface d'onde.

Suivant l'invention, les moyens de réglage de focalisation sont agencés pour forcer le miroir déformable à adopter une courbure supplémentaire, de façon à conjuguer la source lumineuse d'entrée et les moyens de détection avec un point de profondeur prédéterminée dans la rétine, lesdits moyens de réglage étant commandés en synchronisme avec le balayage en Z du montage de tomographie OCT.

Pour restaurer la résolution et le contraste, il importe que l'optique correctrice puisse corriger, dans le bras de l'échantillon, à la fois les fronts d'onde incidents et les fronts d'onde réfléchis. L'optique adaptative doit donc se trouver toute entière dans le bras de l'interféromètre de Michelson menant à l'échantillon, avec sa source de référence et son analyseur.

Dans ces conditions, l'optique correctrice peut, à partir des mesures de front d'onde réalisées par l'analyseur, compenser les perturbations que le front d'onde va rencontrer à sa traversée de l'optique et du milieu environnant l'échantillon. Une image corrigée de chaque point de la source, donc proche de la limite de diffraction, est réalisée dans la profondeur de l'échantillon. Le brouillage de l'information spatiale disparaît et la concentration de lumière augment.

En retour, la même correction est toujours valable pour compenser les aberrations géométriques occasionnées par la traversée du milieu et de l'optique. Une image elle aussi proche de la diffraction est alors disponible et susceptible d'interférer avec le contraste maximum avec son homologue du bras de référence.

Par ailleurs, la mesure du contraste des franges sans modulation par la méthode du Wollaston garantit la cohérence interférométrique des quatre interférogrammes bidimensionnels (2D) nécessaires à la mesure de la carte 2D de réflectance de la rétine, pour une profondeur donnée.

Un second but de l'invention vise à optimiser la sensibilité du dispositif d'OCT. Les aberrations géométriques rencontrées dans l'oeil sur le chemin « aller » dégradent considérablement la conjugaison source Interféromètre de Michelson/Rétine. En conséquence, la résolution latérale de l'éclairement est fortement diminuée. Ces aberrations augmentent très vite avec le diamètre de pupille utilisé. Traditionnellement, la réduction du diamètre du faisceau d'entrée permet de limiter les effets de ces aberrations. Cependant la limite de diffraction augmente et la résolution latérale maximale diminue aussi.

Par ailleurs, les aberrations géométriques rencontrées dans l'oeil sur le chemin « retour » dégradent considérablement la conjugaison Rétine/Détecteur. En conséquence, la résolution latérale de l'image restituée est fortement diminuée. Là aussi, la réduction du diamètre du faisceau de sortie permet de limiter les effets de ces aberrations. Mais là encore, la limite de diffraction augmente et la résolution latérale maximale diminue. De plus, la réduction du diamètre du faisceau s'accompagne d'une réduction de la surface collectrice en sortie de l'oeil, et donc de la sensibilité de l'examen.

En outre, les aberrations géométriques rencontrées dans l'oeil sur le chemin « aller+retour » dégradent considérablement la conjugaison entre les images de la source vues au travers des deux bras de l'interféromètre de Michelson. En conséquence, le contraste interférométrique est fortement diminué. Cet effet n'apparaît que lorsqu'une pupille de trop grand diamètre est utilisée, puisque dans le cas contraire, la réduction du diamètre permet de limiter les aberrations.

Il est remédié à ces inconvénients avec un système de tomographie in vivo selon l'invention comprenant des moyens pour augmenter le contraste interférométrique par la compensation des aberrations géométriques de l'oeil, sous la forme de moyens d'optique adaptative insérés dans le bras de mesure de l'interféromètre, et des moyens pour compenser les effets de biréfringence de la cornée par l'introduction d'un compensateur devant l'oeil.

Les moyens d'optique adaptative, avantageusement réalisés sous la forme d'un miroir déformable, sont installés entre l'interféromètre de Michelson et l'oeil. Ils compensent les aberrations que subit le faisceau en sortant de l'oeil et pré-compensent du même coup les aberrations que subit le faisceau aller en entrant dans l'oeil. L'utilisation de techniques d'optique adaptative dans des ophtalmoscopes est déjà connue de l'art antérieur, notamment par le document EP1164921B1 qui divulgue un système d'optique adaptative mis en oeuvre dans un ophtalmoscope laser à balayage (SLO).

La résolution latérale de l'éclairement est ainsi restaurée. La résolution latérale de l'examen est restaurée. Le contraste interférométrique est optimal, même à pleine ouverture de pupille.

La commande des moyens d'optique adaptative est établie sur la base de mesures de front d'onde réalisées en aval desdits moyens d'optique adaptative (dans le sens du retour) sur l'image d'un point source de référence installé sur la rétine. Ce point source est obtenu par l'introduction d'un faisceau lumineux additionnel, indépendant du faisceau de mesure, focalisé sur la rétine. Suivant le niveau d'optimisation recherché, le faisceau de référence « aller » peut ou non passer par les moyens d'optique adaptative et profiter de la correction adaptative.

La mesure OCT suppose l'égalité des chemins optiques entre les deux bras de l'interféromètre de Michelson, à la longueur de cohérence de la source près. Elle suppose également une mise au point optimale sur la profondeur qui correspond à cette égalité.

Traditionnellement, la limitation du diamètre du faisceau confère à l'oeil une profondeur de champ très grande qui dispense d'une quelconque re-mise au point.

Quand le système est utilisé à pleine ouverture (typiquement F/3), la profondeur de champ diminue rapidement, typiquement 30 µm. Le balayage en Z de l'OCT peut sortir rapidement de cet intervalle, au-delà duquel le contraste interférométrique diminue. On peut considérer cela comme un effet d'aberration de pur défocus.

On peut remédier à ce problème en dotant l'analyseur de surface d'onde d'un dispositif permettant de régler sa propre focalisation, par exemple avec un réglage mécanique. Une modification arbitraire de cette focalisation, force, via la boucle d'optique adaptative, le miroir déformable à adopter une courbure supplémentaire, conjuguant source d'entrée et détecteur avec un point plus ou moins profond dans la rétine. La commande de cette focalisation doit être synchronisée avec le balayage en Z de l'OCT.

Il est aussi possible de commander l'analyseur pour le forcer à travailler en défocalisé. Certains analyseurs évolués, par exemple les modèles de la société Imagine Optic, sont en effet capables de travailler en défocalisé avec de bons résultats.

Une solution alternative à une réelle défocalisation de l'analyseur peut consister à ajouter un terme de focus pur dans la commande du miroir, quelle que soit la mesure de l'analyseur. Cet artifice est couramment utilisé en optique adaptative. Avec un analyseur de type Shack-Hartmann, on modifie simplement le tableau dit « des pentes de référence », ce qui force le système à converger vers une commande arbitrairement modifiée.

Une solution alternative à une réelle défocalisation de l'analyseur peut consister à ajouter un terme de focus pur dans la commande du miroir, quelle que soit la mesure de l'analyseur. Cet artifice est couramment utilisé en optique adaptative. Avec un analyseur de type Shack-Hartmann, on modifie simplement le tableau dit « des pentes de référence », ce qui force le système à converger vers une commande arbitrairement modifiée.

Un autre but de l'invention vise à optimiser le rendement photométrique de l'interféromètre de Michelson mis en oeuvre dans le système de tomographie in vivo selon l'invention. Cet objectif est atteint avec un système de tomographie in vivo selon l'invention opérant en lumière polarisée linéairement et dans lequel on bascule la polarisation entre aller et retour dans les bras avec une lame quart d'onde.

L'emploi d'une lumière polarisée permet :
- de limiter la dégradation du contraste interférométrique par les effets de biréfringence de l'oeil. En effet, toute rotation ou changement de la polarisation de la lumière à son passage dans l'oeil s'accompagne d'une perte du contraste interférométrique par la simple perte de conservation de l'amplitude du champ dans la direction initiale de polarisation ;
- d'optimiser la transmission en retour de l'oeil. En effet, l'emploi d'un cube séparateur polarisant en guide lame séparatrice et la rotation des polarisations dans les deux bras d'un angle de 90 degrés entre aller et retour avec une lame quart d'onde, permet de collecter en sortie de système toute l'énergie renvoyée par l'oeil, et non la moitié, comme c'est traditionnellement le cas avec un interféromètre de Michelson.

Dans une forme particulière de l'invention visant à filtrer au mieux toute réflexion parasite d'origine instrumentale, la lame quart d'onde est placée au plus près de l'oeil, mais avant le compensateur de biréfringence.

On peut avantageusement contribuer à filtrer au mieux la réflexion cornéenne dans le système de tomographie in vivo selon l'invention, en utilisant un montage de Gauss grâce auquel l'adjonction d'un diaphragme de champ stoppe l'essentiel du flux réfléchi par la cornée.

Une optimisation de la mesure du front d'onde peut être avantageusement obtenue en installant la source de référence en amont des moyens compensateurs d'optique adaptative.

Dans le but de ne pas défocaliser la source de référence quand les moyens compensateurs d'optique adaptative, matérialisés à titre d'exemple par un miroir déformable, sont défocalisés pour ajuster la mise au point à une profondeur donnée, on peut en outre prévoir des moyens pour ajuster cette mise au point par réaction des moyens compensateurs d'optique adaptative à une défocalisation globale de l'ensemble source+analyseur.

L'image rétinienne de la source de référence reste ainsi inchangée alors que les moyens compensateurs conjuguent un plan différent de la rétine à la caméra de sortie.

Dans le but d'optimiser la fixation du sujet, on peut avantageusement prévoir une mire active.

Pour garantir la stabilité interférométrique des mesures malgré le caractère adaptatif des corrections de surface d'onde, on peut en outre prévoir des moyens pour geler la forme des moyens compensateurs d'optique adaptative, par exemple du miroir déformable, pendant la durée d'une pose.

Suivant un autre aspect de l'invention, il est proposé un procédé de tomographie in vivo à haute résolution axiale et latérale de la rétine humaine, comprenant :
- une tomographie par interférence à faible longueur de cohérence (OCT) plein champ avec un balayage en Z, mettant en oeuvre une source lumineuse d'entrée,
- une production d'une image de la rétine par des moyens de détection, à partir d'une mesure interférométrique selon le principe de l'OCT,
- une correction des fronts d'onde en provenance de l'oeil et à destination de l'oeil, par des moyens d'optique adaptative, disposés entre l'interféromètre et l'oeil, comprenant une analyse de surface d'onde sur la rétine, et
- un réglage de la focalisation de l'analyse de surface d'onde,
caractérisé en ce que le réglage de focalisation est réalisé de façon à conjuguer la source lumineuse d'entrée et les moyens de détection avec un point de profondeur prédéterminée dans la rétine, en synchronisme avec le balayage en Z de la tomographie OCT.

D'autres avantages et caractéristiques de l'invention apparaîtront à l'examen de la description détaillée d'un mode de mise en oeuvre nullement limitatif, et des dessins annexés sur lesquels :
- la figure 1 est un schéma fonctionnel d'un système de tomographie in vivo selon l'invention ;
- la figure 2 illustre schématiquement la structure de principe d'un système de tomographie in vivo selon l'invention ;
- la figure 3 est un schéma-bloc d'un dispositif de commande d'un système de tomographie in vivo selon l'invention ; et
- la figure 4 est un schéma d'un exemple pratique de réalisation d'un système de tomographie in vivo selon l'invention.
- la figure 5 est un schéma d'un autre exemple de réalisation d'un système de tomographie in vivo selon l'invention.

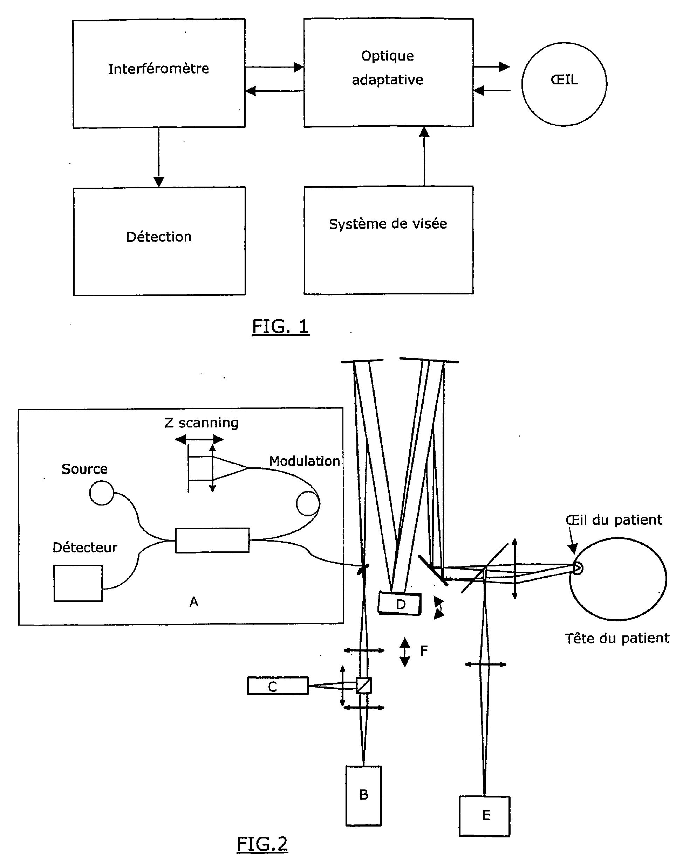

Un système de tomographie in vivo selon l'invention comprend, en référence à la figure 1 :
- un interféromètre de Michelson, réalisant un montage d'OCT plein champ,
- un dispositif d'optique adaptative, disposé entre l'interféromètre et un oeil à examiner, réalisant la correction des fronts d'onde en provenance de l'oeil mais aussi à destination de l'oeil,
- un dispositif de détection, disposé en aval de l'interféromètre, permettant sans modulation ni détection synchrone, de réaliser la mesure interférométrique selon le principe de l'OCT, et
- un système de visée, permettant de guider le regard du patient tout en assurant son confort visuel et en optimisant ses performances de fixation.

On va tout d'abord décrire, en référence à la figure 2, le principe de couplage OCT+OA mis en oeuvre dans un premier exemple de réalisation d'un système de tomographie in vivo selon l'invention. Ce système comprend un système A de tomographie OCT, un analyseur B de front d'onde, une source de référence C, un miroir déformable D assurant également la fonction de balayage de l'oeil sur une ou deux dimensions spatiales, un système de visée E et une lentille F de contrôle de la focalisation.

Le système A de tomographie OCT, de structure conventionnelle, comprend une source, un détecteur, un dispositif de balayage en Z et une modulation.

Une translation de la lentille F permet d'imposer, via la réaction du système adaptatif, une focalisation particulière du miroir déformable D, donc une mise au point particulière. Ce contrôle permet, en association avec le déplacement du miroir de référence, d'observer chaque couche de l'échantillon avec le contraste optimal.

Cette disposition du montage adaptatif complet dans le bras de l'interféromètre de Michelson rend la correction de front d'onde possible.

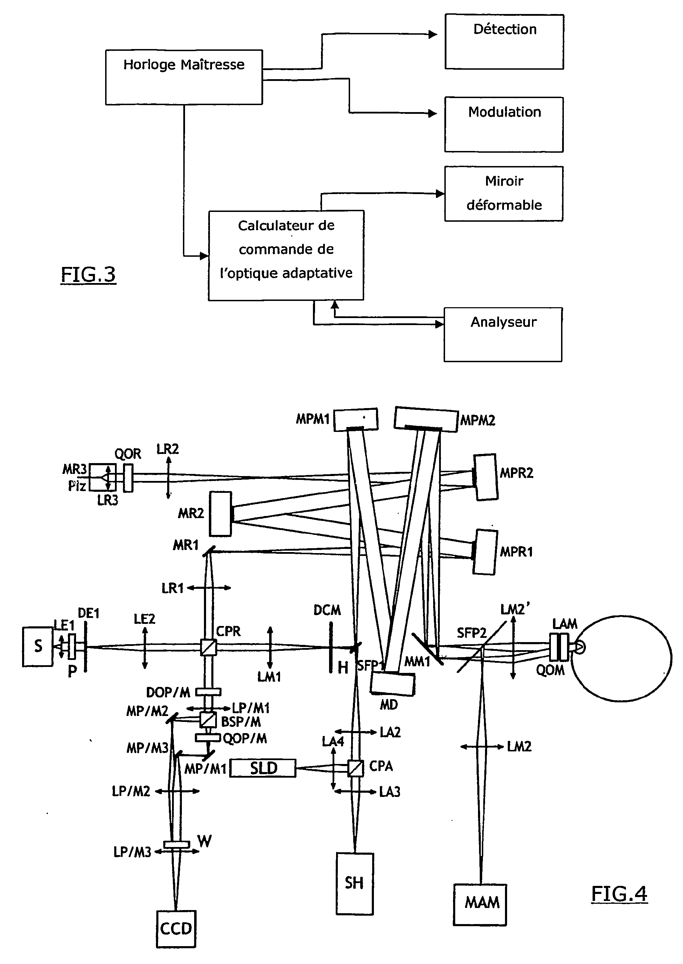

Les systèmes OCT utilisés actuellement sur des tissus biologiques tels qu'un oeil, extraient un faible signal interférométrique disponible du fond incohérent, à l'aide de techniques de détection synchrone associées à une modulation du chemin optique, le plus souvent par un changement de la longueur de la fibre sur le bras de référence. Cette modulation doit être ici synchronisée avec les fronts d'onde de manière à ce qu'aucune différence de marche additionnelle d'origine 'adaptative' ne vienne perturber la mesure interférométrique. Au-delà, c'est-à-dire sur des intervalles de temps plus longs que la période de modulation/démodulation du système, la différence de marche doit également être maintenue constante à la moitié de la longueur de cohérence près, puisque c'est in fine cette dernière qui fixe la résolution en profondeur du système. Un dispositif de commande, tel qu'illustré schématiquement sur la figure 3, est programmé pour assurer ce contrôle de la différence de marche. Ce dispositif de commande peut comprendre une horloge maîtresse délivrant un signal d'horloge à unité de détection, à une unité de modulation et à un calculateur de commande de l'optique adaptative qui pilote un miroir déformable et un analyseur.

On va maintenant décrire, en référence à la figure 4, un exemple pratique de réalisation d'un système de tomographie in vivo selon l'invention. L'interféromètre, de type Michelson, comprend un bras de mesure prévu pour illuminer l'oeil et collecter la lumière renvoyée, et un bras de référence prévu pour illuminer un miroir mobile permettant l'exploration en profondeur du tissu rétinien.

L'interféromètre est utilisé en lumière polarisée de façon rectiligne et perpendiculaire dans les deux bras. La source de lumière S est une diode à faible longueur de cohérence temporelle (par exemple, 12 µm), dont le spectre est centré sur 780 nm. Elle confère par principe au système de tomographie in vivo une résolution axiale égale à la moitié de la longueur de cohérence divisée par l'indice de réfraction du milieu.

Cette source de lumière S peut être pulsée. Dans ce cas, elle est alors synchronisée avec la prise d'image et la correction adaptative. Le faisceau est limité par un diaphragme de champ correspondant à 1 degré dans le champ de vue de l'oeil (300 µm sur la rétine) et un diaphragme pupillaire correspondant à une ouverture de 7 mm sur un oeil dilaté.

Un polariseur d'entrée P permet l'équilibrage optimal des flux injectés dans les deux bras de l'interféromètre.

Les deux bras présentent une configuration dite de Gauss, afocale, qui permet le transport des pupilles, d'une part, et la matérialisation d'une image intermédiaire du champ où un diaphragme bloque une grande part du reflet cornéen, d'autre part. Des lames quart d'onde assurent par la rotation de la polarisation de la seule lumière renvoyée par l'oeil, et le miroir mobile, un filtrage efficace des réflexions parasités dans le système de tomographie in vivo selon l'invention.

Afin de conserver l'égalité des chemins optiques dans les deux bras, avec le même transport des pupilles et du champ, le bras de référence est similaire au bras de mesure, mais avec un optique statique.

On va maintenant décrire la voie de détection du système de tomographie in vivo selon l'invention. Les deux faisceaux sur le bras de sortie sont encore polarisés perpendiculairement, et ils n'interfèrent que s'ils sont projetés sur une direction commune. Un prisme de Wollaston W a pour fonction de projeter simultanément les deux rayonnements sur deux directions d'analyse perpendiculaires. On peut alors effectuer une mesure simultanée de l'intensité après interférence dans deux états d'interférence en opposition, sans modulation ni détection synchrone, sur un détecteur bidimensionnel unique. L'adjonction d'une lame quart d'onde, après division du faisceau, permet d'accéder à deux mesures supplémentaires, levant ainsi toute ambiguïté entre amplitude et phase des franges. Une lame demi onde à l'entrée de la voie de détection ou devant le prisme de Wollaston permet d'orienter convenablement les polarisations incidentes.

Le prisme de Wollaston est placé dans un plan pupillaire, donc conjugué du cube séparateur de l'interféromètre de Michelson. L'angle de séparation du prisme de Wollaston est choisi en fonction du champ à observer. La longueur focale de l'objectif final détermine le pas d'échantillonnage des quatre images.

Le détecteur est du type CCD, avec une cadence d'image supérieure à 30 images par seconde. Ce détecteur est associé à un calculateur dédié (non représenté) dans lequel es réalisé le traitement numérique des images : extraction des quatre mesures, étalonnage, calcul de l'amplitude des franges.

La correction adaptative des fronts d'onde est réalisée en amont de l'interféromètre, donc dans le bras de mesure. Chaque point de la source S voit ainsi son image sur la rétine corrigée des aberrations, et l'image en retour est également corrigée. L'amplitude des franges est alors maximale.

Le sous-ensemble d'optique adaptative comprend un miroir déformable MD. La mesure de front d'onde est faite par un analyseur SH de type Shack-Hartmann sur le faisceau de retour d'un spot lumineux lui-même imagé sur la rétine via le miroir déformable MD. La longueur d'onde d'analyse est de 820 nm. L'éclairage est continu et fourni par une diode SLD superluminescente temporellement incohérente. Le dimensionnement de l'analyseur correspond à une optimisation entre sensibilité photométrique et échantillonnage du front d'onde. La cadence de rafraîchissement de la commande du miroir déformable MD peut atteindre 150 Hz. Un calculateur dédié (non représenté) gère la boucle d'optique adaptative. La commande est toutefois synchronisée pour geler la forme du miroir pendant la mesure interférométrique.

Un contrôle approprié de la focalisation de la voie d'analyse, au moyen d'une lentille LA2, permet d'adapter la distance de focalisation à la couche sélectionnée par l'interféromètre. Cette disposition est capitale pour conserver un contraste optimal à toute profondeur.

Le miroir déformable MD est conjugué de la pupille du système et de l'oeil. Le champ du système est défini par le diaphragme de champ DCM d'entrée du système. Il est choisi égal à 1 degré, soit moins que le champ d'isoplanétisme de l'oeil, ce qui garantit la validité de la correction adaptative dans le champ sur la seule mesure de front d'onde réalisée à partir du spot, au centre du champ. De plus, la rotation du miroir déformable MD permet de choisir l'angle d'arrivée du faisceau dans l'oeil, donc la portion de rétine étudiée.

L'adjonction de verres correcteurs de la vue du sujet, donc des bas ordres d'aberrations géométriques tels que le focus ou l'astigmatisme, juste devant l'oeil, permet de relâcher les exigences sur la course du miroir déformable MD, et garantit également une meilleure visée. Un système correcteur adaptatif par transmission peut être utilisé de préférence à des verres fixes pour une correction optimale.

Un système de visée collaboratif ou actif est installé en amont de l'ensemble. Ce système de visée, qui comprend une mire active MAM, présente au sujet l'image d'un point lumineux s'écartant périodiquement de l'axe de visée recherché. Le patient est alors invité à suivre tous les mouvements de cette image. Chaque fois que l'image revient sur l'axe, et après un temps de latence ajustable, une série de mesures interférométriques est réalisée. Le déplacement périodique du regard permet d'obtenir du patient une meilleure capacité de fixation quand il vise l'axe recherché. L'amplitude et la fréquence sont adaptables au sujet et aux mesures entreprises. Pour des raisons de commodité, la mire peut être réalisée avec un simple ordinateur de bureau sur lequel un point lumineux est affiché et déplacé. La mire active MAM, l'optique adaptative, la source S et la prise d'image sont synchronisées.

On va maintenant décrire de façon détaillée, en référence à la figure 4, un exemple concret de réalisation de chacun des sous-ensembles d'un système de tomographie in vivo selon l'invention.

Le bras d'entrée comprend successivement la source S, typiquement une diode électroluminescente d'une puissance de 30 mW avec une longueur d'onde de 780 nm, de modèle Hitachi HE7601SG, un condenseur LE1 réalisé sous la forme d'un objectif de microscope, un diaphragme de champ DE1, et un collimateur LE2.

Le bras de référence comprend successivement une séparatrice CPR réalisée sous la forme d'un cube polarisant large bande, une première lentille de Gauss LR1 de focale 125 mm, un premier miroir MR1 de renvoi des faisceaux, un premier miroir parabolique MPR1 de renvoi parabolique des faisceaux, un second miroir MR2 de renvoi des faisceaux, un second miroir MPR2 de renvoi parabolique des faisceaux, une seconde lentille de Gauss LR2, une lame quart d'onde QOR réalisant une fonction de rotation de polarisation, une troisième lentille LR3 de focalisation et un dernier miroir MR3 sphérique et de courbure centrée sur le centre de la lentille LR3. Ce miroir MR3 assure à la fois la fonction de rétine de référence de l'interféromètre et de reconjugaison de la pupille en retour.

Le bras de mesure comprend une première lentille de Gauss LM1, un diaphragme de champ DCM, un séparateur de faisceaux SFP1, une seconde lentille de Gauss LM2, une séparatrice CPA sous la forme d'un cube polarisant large bande, une lentille de focalisation LA3, un analyseur de Shack-Hartmann SH, une lentille de focalisation LA4, une source d'analyse SLD à une longueur d'onde de 830 nm, un premier miroir parabolique MPM1 de renvoi parabolique des faisceaux, un miroir déformable MD comportant 31 électrodes, un second miroir parabolique MPM2 de renvoi parabolique des faisceaux, un miroir MM1 de renvoi des faisceaux, un séparateur pelliculaire SFP2, une lentille de Gauss LM2', une lame quart d'onde QOM de rotation de polarisation, une lentille adaptative LAM, une lentille de focalisation LM2 et une mire active MAM.

Le bras d'imagerie comprend une lame demi-onde DOP/M prévue pour effectuer un basculement des polarisations, un cube séparateur non polarisant BSP/M, un miroir MP/M1 de renvoi des faisceaux, une lame quart d'onde QOP/M de création d'un retard différentiel fonction des polarisations sur l'un des faisceaux, un miroir MP/M2 de renvoi des faisceaux, un miroir MP/M3, une lentille LP/M2 de collimation, un prisme de Wollaston W de projection des polarisations, une lentille LP/M3 d'objectif, et un détecteur CCD. La lame demi-onde peut avantageusement être placée juste avant le prisme de Wollaston, auquel cas les polarisations voyagent dans le bras de mesure avec l'orientation qu'elles ont dans l'interféromètre (en retour). La lame quart d'onde du bras de mesure doit donc être orientée en conséquence.

Dans l'exemple pratique de réalisation illustré par la figure 4, le système de tomographie in vivo selon l'invention est relativement compact, moins de 1,2 m de côté. Une part importante de la contrainte de taille vient du diamètre du miroir déformable MD qui fixe en partie la longueur focale des paraboles hors axe. L'emploi de micro-miroirs diminuerait évidemment toutes les dimensions du système.

Le système de détection, avec sa division en deux faisceau, est réalisé ici avec des composants discrets. Il est envisageable de faire réaliser et d'utiliser des composants intégrés réunissant les fonctions de séparation, repliement, voire, retard des faisceaux.

On va maintenant exposer les performances techniques du système de tomographie in vivo selon l'invention, dans sa configuration pratique représentée en figure 4. A chaque pose, une image plein champ de la rétine est réalisée, avec un diamètre de 1 degré. L'image est corrigée des aberrations géométriques, donc proche de la limite de diffraction (1,8 microns à 780 nm de longueur d'onde). Le caractère simultané, car plein champ, des mesures en tout point confère à l'image une précision cartographique maximale. Par ailleurs, le caractère simultané de la mesure interférométrique garantit la conservation de la résolution en profondeur : 4,5 microns avec une source de 12µm de longueur de cohérence.

Ainsi l'emploi conjugué d'une technique interférométrique plein champ, d'une optique adaptative en amont, et d'une détection simultanée sans modulation, rend possible l'obtention d'images tridimensionnelles in vivo. Par ailleurs, la mire active optimise la performance opérationnelle du système.

Le caractère plein champ du dispositif OCT utilisée dans le système de tomographie in vivo selon l'invention procure un gain en sensibilité de nature multiplex, à savoir un gain obtenu en parallélisant l'intégration du flux lumineux des différents points du champ, par comparaison à toute technique de balayage. L'emploi d'une optique adaptative garantit non seulement la restauration de la qualité d'image mais aussi celle du contraste interférométrique. En ce qui concerne l'analyse de front d'onde, on notera que l'installation de la source de référence en amont du miroir déformable garantit une qualité de mesure des aberrations optimale puisque l'image de référence matérialisée dans l'oeil bénéficie de la correction adaptative. Cette optimisation reste vraie quelle que soit la focalisation, la source étant en amont du système de contrôle de mise au point. Enfin, l'emploi d'un cube polarisant permet là encore d'utiliser tous les photons issus de l'oeil. La mesure du front d'onde est donc faite dans les meilleures conditions.

Le champ instantané accessible au système, donc sur une pose, est d'environ un degré, soit 300 µm sur la rétine. Si le dispositif de balayage en profondeur est mis en oeuvre entre chaque prise d'image, en 1 seconde, jusqu'à 30 plans de tissu peuvent être examinés. Si le patient peut maintenir sa fixation pendant 2 secondes, et si la distance entre les plans est choisie égale à 2 µm, par exemple, un volume de 300x300x120 µm de tissu rétinien peut ainsi être observé avec une résolution de 1,8x1,8x4,5 microns.

La possibilité de rotation du miroir déformable apporte par ailleurs au système de tomographie in vivo selon l'invention la capacité d'explorer un champ plus large, sur plusieurs degrés, donc d'examiner des régions plus périphériques de la zone fovéolaire.

En ce qui concerne l'analyse de front d'onde, on notera que l'installation de la source de référence SLD en amont du miroir déformable MD permet une qualité optimale de mesure des aberrations donc de leur compensation, puisque l'image de référence matérialisée dans l'oeil bénéficie dans ce cas de la correction adaptative. Cette optimisation reste vraie quelle que soit la focalisation, la source SLD étant en amont du système de contrôle de mise au point. Enfin, l'emploi d'un cube polarisant CPA (fig. 4) permet là encore d'utiliser tous les photons issus de l'oeil. La mesure du front d'onde est donc faite dans de bonnes conditions.

Cependant, il est à noter qu'une conjugaison optique latérale très précise est requise entre la source de référence SLD et l'entrée de l'analyseur SH. Dans le cas où cette conjugaison est insuffisante, une tentative de compensation par la boucle d'optique adaptative d'une erreur de conjugaison peut mener à une divergence de l'asservissement.

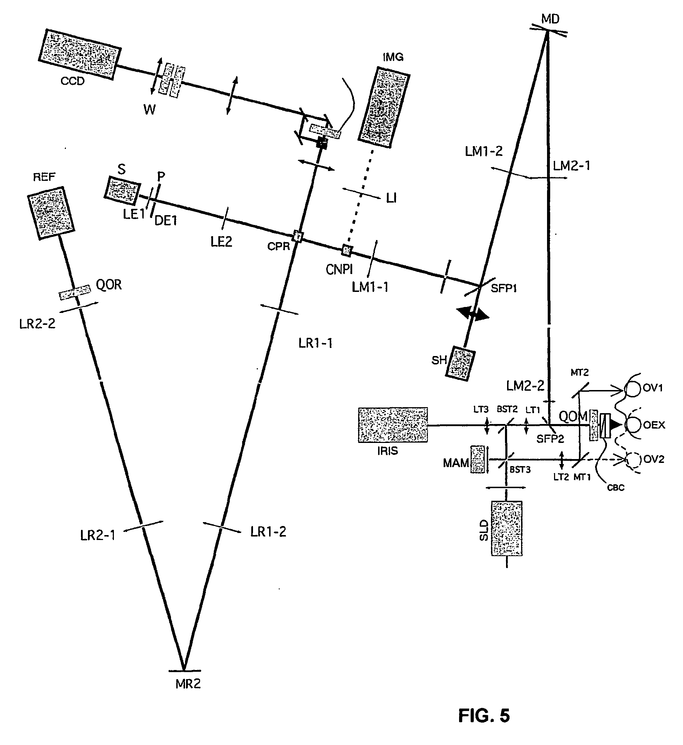

Une variante de l'invention, illustrée en figure 5, permet plus de simplicité dans le système en diminuant ce risque d'instabilité de la boucle d'optique adaptative. Dans cette variante, la source de référence SLD est positionnée plus près de l'oeil dans le trajet optique, en particulier après l'optique adaptative (à l'aller) et par exemple avant un compensateur de biréfringence, comme un compensateur de Soleil-Babinet CBC, ou juste avant l'oeil. A défaut de bénéficier d'une tache image optimale au fond de l'oeil, le système gagne alors en stabilité de fonctionnement.

Le miroir déformable utilisé dans l'optique adaptive peut être par exemple un miroir de diamètre 50 mm à 31 éléments de la société CILAS. Cependant, les performances et/ou la compacité du dispositif peuvent être améliorées en utilisant un modèle plus performant et/ou plus compact tel que le miroir déformable de diamètre 15 mm à 52 éléments développé au Laboratoire d'Astrophysique de l'Observatoire de Grenoble, en particulier du fait de sa compacité et d'une course plus importante dans les mouvements d'adaptation.

Dans l'exemple illustré en figure 5, les miroirs de repliement du bras de mesure MPM1, MPM2 et ceux du bras de référence MPR1, MPR2 ont été supprimés. Le trajet optique du bras de mesure comprend un doublet de deux lentilles LM1-1 et LM1-2 d'un côté du miroir déformable MD, et un autre doublet de deux lentilles LM2-1 et LM2-2 de l'autre côté de ce miroir déformable. De la même façon, le trajet optique du bras de référence comprend un doublet de deux lentilles LR1-1 et LR1-2 d'un côté du miroir de référence MR, et un autre doublet de deux lentilles LR2-1 et LR2-2 de l'autre côté de ce miroir de référence.

L'utilisation de lentilles plutôt que de miroirs peut être plus économique et permettre de meilleures performance, en particulier du fait du coût et des aberrations optiques de ce type de miroir, qui sont typiquement des miroirs paraboliques hors axes.

La combinaison d'un tel montage dans l'axe, avec un miroir déformable plus petit permet d'obtenir un système plus performant, plus simple, ou plus économique, tout en conservant un encombrement limité.

Ainsi qu'illustré en figure 5, le système peut en outre comprendre des moyens d'imagerie classique, comme une caméra IMG, permettant d'associer les mesures interférométriques avec une imagerie simple des zones examinées, par exemple pour faciliter l'exploration et la sélection des zones à examiner.

Placé directement en sortie (au retour) du bras de mesure, donc juste avant le cube polarisant CPR de l'interféromètre, un second cube polarisant CNPI permet de dévier le faisceau de retour vers une caméra d'imagerie IMG disposant de ses propres moyens de focalisation LI de l'image. Sur cette voie, une image directe de la zone rétinienne visée sera observable. On peut en particulier agencer le bras de mesure et cette voie additionnelle de sorte qu'ils procurent un champ d'observation plus large que le mode interférométrique, dont le champ est limité en particulier par la technique de mesure de contraste interférométrique en elle-même.

Du fait de sa faible longueur de cohérence, la source d'entrée S présente un spectre de type polychromatique. Dans un montage OCT typique ce spectre est en général relativement étroit, par exemple d'une largeur de l'ordre de 50 nanomètres, mais pas forcément négligeable.

Ce spectre polychromatique peut causer une dégradation des performances, en particulier en entraînant une dispersion des différences de marche du fait du caractère dispersif du milieu oculaire, ce qui conduit à une dégradation de la résolution axiale du dispositif. Pour éviter ou limiter ces dégradations, le système peut comprendre des moyens de compensation situés dans le bras de référence.

De plus, le caractère dispersif des milieux oculaires se traduit également par une variation de la focale de l'oeil avec la longueur d'onde, entraînant aussi une dégradation de la résolution axiale. Pour éviter ou limiter ces dégradations, le système peut alors comprendre des moyens de compensation situés par exemple dans le bras de mesure. En particulier, ces moyens peuvent compenser un chromatisme focal qui représente environ 400 micromètres entre le rouge et le bleu, par exemple en remplaçant le collimateur LM2-2 situé juste devant l'oeil par un doublet au chromatisme volontairement choisi opposé à celui de l'oeil. Ces moyens peuvent également compenser les différences de chemin optique dues à la dispersion chromatique, par exemple en insérant une cuve d'eau dans le bras de référence d'une taille dépendant et/ou réglable selon la taille ou les caractéristiques de l'oeil à examiner. Une telle cuve peut être d'une dimension de l'ordre de 24 mm, longueur moyenne d'un oeil humain.

Avec une source de 12 micromètres de longueur de cohérence et de 50 nanomètres de largeur de spectre, l'utilisation de ces moyens de compensation peut permettre d'améliorer la résolution axiale en la ramenant d'une valeur d'environ 6 micromètres à une valeur d'environ 4 micromètres.

Pour augmenter les performances, en particulier en terme de résolution axiale, le système peut également utiliser comme source d'entrée de l'interféromètre une illumination polychromatique à spectre plus large, par exemple en lumière blanche. Dans ce cas, l'augmentation de performance procurée par ces moyens de compensation sera beaucoup plus importante.

Dans l'exemple illustré en figure 5, le système est agencé pour que la cible de la mire active MAM soit visible par les deux yeux OD1 et OG1 du sujet à examiner. Une visée avec les deux yeux peut en effet permettre d'améliorer les performances de fixations ou de stabilité, et faciliter l'examen. Dans cet exemple, l'image de la mire est introduite dans le trajet optique entre la source de référence SLD et l'oeil examiné par une séparatrice BST3.

Cette séparatrice peut être choisie dichroïque de manière à réfléchir 50% de toute la lumière venant de la mire MAM vers l'oeil examiné OEX, et transmettre les 50% restant vers l'autre oeil OV1 ou OV2 pour permettre une visée des deux yeux. La séparatrice dichroïque BST3 transmet alors toute la lumière de la source de référence SLD vers l'oeil examiné OEX, en profitant d'une différence de spectre entre la source de référence SLD (830 nm) et la mire MAM (800 nm). Une lame séparatrice 50/50 totalement neutre spectralement convient également, mais 50% de la lumière de la SLD est alors envoyé vers l'oeil qui n'est pas étudié. Un filtre peut permettre d'éliminer cette image si elle est jugée gênante par le sujet.

De façon à pouvoir examiner n'importe lequel des deux yeux tout en assurant une visée des deux yeux, le système présente un emplacement central d'examen OEX, ainsi que deux emplacements de visée OV1 et OV2 répartis des deux côtés de cet emplacement d'examen OEX.

Lorsque l'oeil gauche est à l'emplacement central pour être examiné, l'oeil droit reçoit l'image de la mire MAM dans son emplacement de visée OV1 par des moyens de renvoi escamotables, par exemples deux miroirs MT1 et MT2. Lorsque c'est l'oeil droit qui est à l'emplacement d'examen OEX, les moyens de renvoi peuvent être escamotés ou annulés et l'image de la mire MAM parvient à l'oeil gauche dans son emplacement de visée OV2.

Ainsi qu'illustré en figure 5, le système peut également comprendre, ou collaborer avec, des moyens de suivi IRIS des mouvements de l'oeil à examiner, collaborant avec le dispositif de tomographie. Il peut s'agir par exemple d'une caméra avec reconnaissance d'image réalisant un suivi ou « tracking », par exemple de la rétine ou de pupille ou des bords de l'iris, de façon à détecter et évaluer les mouvements de l'oeil.

La connaissance des mouvements de l'oeil permet alors au système de s'adapter aux déplacements de la zone à examiner, par exemple en coordonnant et les réglages et les prises de vue avec les différentes positions détectées ou prévues de cette zone à examiner, ou en permettant une optimisation spatiale et/ou temporelle de l'optique adaptive. Il est possible par exemple de profiter des périodes naturelles de stabilisation de la pupille ou de la rétine pour réaliser tout ou partie des réglages ou des mesures souhaités.

L'image de l'oeil examiné parvient aux moyens de suivi de l'oeil IRIS par une séparatrice BST2 insérée dans le trajet optique, par exemple entre l'oeil et la source de référence SLD. De façon avantageuse, par exemple pour ne pas gêner le sujet, cette séparatrice BST2 est dichroïque et le suivi des mouvements de l'oeil se fait en lumière non visible, par exemple infrarouge.

Les moyens de suivi IRIS peuvent comprendre par exemple un dispositif de mesure des déplacements oculaires, comme ceux développés par la société Métrovision.

L'invention peut en particulier être mise en oeuvre pour réaliser ou compléter un dispositif d'imagerie rétinienne, ou de topographie cornéenne, ou de mesure d'un film de larmes.

Bien sûr, l'invention n'est pas limitée aux exemples qui viennent d'être décrits et de nombreux aménagements peuvent être apportés à ces exemples sans sortir du cadre de l'invention.

## Revendications

1. Système de tomographie in vivo à haute résolution axiale et latérale de la rétine humaine, comprenant :
- un interféromètre de Michelson, réalisant un montage de tomographie par interférence à faible longueur de cohérence (OCT) plein champ avec un balayage en Z,
- une source lumineuse d'entrée (S) disposée en un bras d'entrée de l'interféromètre,
- des moyens d'optique adaptative prévus pour corriger des fronts d'onde en provenance de l'oeil et à destination de l'oeil, comprenant une source de référence (SLD), un miroir déformable (MD) et des moyens d'analyse de surface d'onde (SH),
- des moyens de détection (CCD), disposés en un bras d'imagerie de l'interféromètre, prévus pour produire une image à partir d'une mesure interférométrique selon le principe de l'OCT, et
- des moyens (LA2, LA3, LA4) pour régler la focalisation des moyens d'analyse de surface d'onde (SH),
**caractérisé en ce que** les moyens de réglage de focalisation sont agencés pour forcer le miroir déformable à adopter une courbure supplémentaire, de façon à conjuguer la source lumineuse d'entrée (S) et les moyens de détection (CCD) avec un point de profondeur prédéterminée dans la rétine, lesdits moyens de réglage étant commandés en synchronisme avec le balayage en Z du montage de tomographie OCT.

2. Système selon la revendication 1, **caractérisé en ce que** les moyens d'optique adaptative (MD, SLD, SH) sont disposés entre l'interféromètre de Michelson et l'oeil à examiner (OEX).

3. Système selon l'une des revendications 1 ou 2, **caractérisé en ce qu'**il comprend en outre des moyens pour commander les moyens d'optique adaptative (MD) sur la base de mesures de front d'onde réalisées en aval desdits moyens d'optique adaptative sur une image ponctuelle de la source de référence (SLD) réalisée sur la rétine de l'oeil.

4. Système selon la revendication 3, **caractérisé en ce qu'**il comprend en outre des moyens pour introduire un faisceau lumineux additionnel, indépendant du faisceau de mesure, focalisé sur la rétine.

5. Système selon l'une des revendications précédentes, **caractérisé en ce que** les moyens d'analyse de surface d'onde (SH) comprennent un analyseur de type Shack-Hartmann.

6. Système selon l'une des revendications précédentes, **caractérisé en ce qu'**il comprend en outre des moyens (CBC) pour compenser les effets de biréfringence de la cornée, qui sont disposés devant l'oeil (OEX).

7. Système selon l'une quelconque des revendications précédentes, **caractérisé en ce que** les deux bras de l'interféromètre sont parcourus par de la lumière polarisée rectilignement.

8. Système selon la revendication 7, **caractérisé en ce qu'**il comprend en outre un cube polarisant (CPR) pour obtenir dans chaque bras deux polarisations perpendiculaires entre elles.

9. Système selon la revendication 8, **caractérisé en ce que** les deux bras de l'interféromètre comprennent des moyens pour faire basculer de 90 degrés la polarisation entre l'aller et le retour.

10. Système selon la revendication 9, **caractérisé en ce que** les moyens de basculement de polarisation comprennent une lame quart d'onde (QOR, QOM).

11. Système selon l'une des revendications 7 à 10, **caractérisé en ce que** l'interféromètre est illuminé en lumière polarisée linéairement (S, P).

12. Système selon l'une des revendications 7 à 11, **caractérisé en ce qu'**il comprend en outre des moyens pour ajuster l'orientation de la polarisation rectiligne d'entrée (P), de façon à obtenir une répartition prédéterminée des flux injectés dans les deux bras de l'interféromètre.

13. Système selon les revendications 6 et 10, **caractérisé en ce que** la lame quart d'onde (QOM) est placée au plus près de l'oeil, avant les moyens de compensation de biréfringence.

14. Système selon l'une des revendications précédentes, **caractérisé en ce qu'**il comprend en outre des moyens pour filtrer la réflexion cornéenne.

15. Système selon la revendication 14, **caractérisé en ce que** les moyens de filtrage de la réflexion cornéenne comprennent un diaphragme de champ (DCM) disposé pour stopper l'essentiel du flux réfléchi par la cornée.

16. Système selon l'une des revendications précédentes, **caractérisé en ce qu'**il comprend en outre des moyens pour ajuster la mise au point à une profondeur donnée, par réaction des moyens d'optique adaptative (MD) à une défocalisation globale de l'ensemble constitué par la source de référence (SLD) et les moyens analyseurs (SH).

17. Système selon l'une des revendications précédentes, **caractérisé en ce qu'**il comprend en outre une mire active (MAM).

18. Système selon l'une quelconque des revendications précédentes, **caractérisé en ce qu'**il comprend en outre des moyens pour geler la forme des moyens d'optique adaptative (MD) pendant la durée d'une pose.

19. Système selon l'une des revendications précédentes, **caractérisé en ce que** la source de référence (SLD) est disposée en amont des moyens d'optique adaptative (MD).

20. Système selon l'une des revendications 1 à 18, **caractérisé en ce que** la source de référence (SLD) est insérée dans le chemin optique entre les moyens d'optique adaptative (MD) et l'oeil à examiner (OEX).

21. Système selon l'une des revendications précédentes, **caractérisé en ce qu'**il comprend des moyens (IRIS) de suivi du mouvement de l'oeil à examiner collaborant avec les moyens de réglage ou de détection.

22. Système selon l'une des revendications précédentes, **caractérisé en ce qu'**il comprend, dans le bras de mesure, des moyens de compensation des effets du chromatisme focal de l'oeil.

23. Système selon l'une des revendications précédentes, **caractérisé en ce qu'**il comprend dans le bras de référence des moyens de compensation de la dispersion des différences de marche.

24. Procédé de tomographie in vivo à haute résolution axiale et latérale de la rétine humaine, comprenant :
- une tomographie par interférence à faible longueur de cohérence (OCT) plein champ avec un balayage en Z, mettant en oeuvre une source lumineuse d'entrée (S),
- une production d'une image de la rétine par des moyens de détection (CCD), à partir d'une mesure interférométrique selon le principe de l'OCT,
- une correction des fronts d'onde en provenance de l'oeil et à destination de l'oeil, par des moyens d'optique adaptative (MD, SLD, SH), disposés entre l'interféromètre et l'oeil, comprenant une analyse de surface d'onde sur la rétine, et
- un réglage de la focalisation de l'analyse de surface d'onde,
- **caractérisé en ce que** le réglage de focalisation est réalisé de façon à conjuguer la source lumineuse d'entrée (S) et les moyens de détection (CCD) avec un point de profondeur prédéterminée dans la rétine, en synchronisme avec le balayage en Z de la tomographie OCT.

25. Procédé selon la revendication 24, **caractérisé en ce que** la mesure interférométrique comprend une mesure du contraste des franges sans modulation par la méthode dite du Wollaston.

26. Procédé selon l'une des revendications 24 ou 25, **caractérisé en ce qu'**il comprend en outre une compensation des effets de biréfringence de la cornée.

27. Procédé selon la revendication 26, **caractérisé en ce qu'**il comprend en outre une polarisation linéaire (CPA) de la source de référence (SLD) et un basculement de la polarisation entre aller et retour dans les bras.

28. Procédé selon l'une des revendications 24 à 27, **caractérisé en ce qu'**il comprend en outre un filtrage (DCM) de la réflexion cornéenne.

29. Procédé selon l'une des revendications 24 à 28, **caractérisé en ce qu'**il comprend en outre un ajustement de la mise au point à une profondeur donnée, en commandant l'optique adaptative (MD) en réaction à une défocalisation globale de l'ensemble constitué par la source de référence (SLD) et les moyens analyseurs de surface d'onde (SH).

30. Procédé selon l'une des revendications 24 à 29, **caractérisé en ce qu'**il comprend en outre un réglage de la focalisation des moyens analyseurs de surface d'onde (SH).

31. Procédé selon l'une des revendications 24 à 30, **caractérisé en ce qu'**il comprend en outre un gel de la forme des moyens d'optique adaptative (MD) pendant la durée d'une pose.

32. Procédé selon l'une des revendications 24 à 31, **caractérisé en ce qu'**il comprend, dans le bras de mesure, une compensation des effets du chromatisme focal de l'oeil.

33. Procédé selon l'une des revendications 24 à 32, **caractérisé en ce qu'**il comprend, dans le bras de référence, des moyens de compensation de la dispersion des différences de marche.

34. Procédé selon l'une des revendications 24 à 33, **caractérisé en ce qu'**il comprend une commande de l'analyseur de front d'onde (SH) l'obligeant à travailler en défocalisé.

## Claims

1. *In vivo* tomography system with high axial and lateral resolution of the human retina, comprising:
- a Michelson interferometer, producing a full-field tomography setup by interference with low coherence length (OCT) with a Z scanning,
- an input light source (S) arranged in an input arm of the interferometer,
- adaptive optical means, designed to correct wavefronts originating from the eye and directed to the eye, comprising a reference source (SLD), a deformable mirror (MD) and means of analysing the wave surface (SH),
- means of detection (CCD), arranged in an imaging arm of the interferometer, designed to produce an image from an interferometric measurement according to the OCT principle, and
- means of adjusting (LA2, LA3, LA4) the focussing of the means of analysis of the wave surface (SH),
**characterized in that** the means of adjusting the focussing are arranged to force the deformable mirror to adopt an additional curvature, so as to conjugate the input light source (S) and the means of detection (CCD) with a point at a predetermined depth in the retina, said means of adjustment being controlled in synchronism with the Z scanning of the OCT tomography setup.

2. System according to claim 1, **characterized in that** the adaptive optical means (MD, SLD, SH) are arranged between the Michelson interferometer and the eye to be examined (OEX).

3. System according to one of claims 1 or 2, **characterized in that** it further comprises means for controlling the adaptive optical means (MD) based on wavefront measurements made downstream of said adaptive optical means on a point image of the reference source (SLD) produced on the retina of the eye.

4. System according to claim 3, **characterized in that** it also comprises means to introduce an additional light beam, independent of the measurement beam, focussed on the retina.

5. System according to one of the preceding claims, **characterized in that** the means of analysing the wave surface (SH) comprise an analyser of the Shack-Hartmann type.

6. System according to one of the preceding claims, **characterized in that** it also comprises means (CBC) to compensate for the effects of birefringence of the cornea, which are arranged in front of the eye (OEX).

7. System according to any one of the preceding claims, **characterized in that** rectilinearly polarized light passes through the two arms of the interferometer.

8. System according to claim 7, **characterized in that** it further comprises a polarizing cube (CPR) in order to obtain two mutually perpendicular polarizations in each arm.

9. System according to claim 8, **characterized in that** the two arms of the interferometer comprise means to switch the polarization by 90 degrees between the outward and return paths.

10. System according to claim 9, **characterized in that** the means of switching the polarization comprise a quarter-wave plate (QOR, QOM).

11. System according to one of claims 7 to 10, **characterized in that** the interferometer is illuminated with linearly polarized light (S, P).

12. System according to one of claims 7 to 11, **characterized in that** it also comprises means of adjusting the orientation of the input rectilinear polarization (P), so as to obtain a predetermined division of the fluxes injected into the two arms of the interferometer.

13. System according to claims 6 and 10, **characterized in that** the quarter-wave plate (QOM) is placed closest to the eye, before the birefringence compensation means.

14. System according to one of the preceding claims, **characterized in that** it further comprises means of filtering the corneal reflection.

15. System according to claim 14, **characterized in that** the means of filtering the corneal reflection comprise a field diaphragm (DCM) arranged to stop the essential part of the flux reflected by the cornea.

16. System according to one of the preceding claims, **characterized in that** it further comprises means of tuning the adjustment to a given depth, through reaction of the adaptive optical means (MD) to an overall defocusing of the assembly constituted by the reference source (SLD) and the analyser means (SH).

17. System according to one of the preceding claims, **characterized in that** it further comprises an active sighting target pattern (MAM).

18. System according to any one of the preceding claims, **characterized in that** it further comprises means of freezing the shape of the adaptive optical means (MD) for the duration of an exposure.

19. System according to one of the preceding claims, **characterized in that** the reference source (SLD) is arranged upstream of the adaptive optical means (MD).

20. System according to one of claims 1 to 18, **characterized in that** the reference source (SLD) is inserted into the optical path between the adaptive optical means (MD) and the eye to be examined (OEX).

21. System according to one of the preceding claims, **characterized in that** it comprises means (IRIS) for tracking the movement of the eye to be examined collaborating with the means of adjustment or detection.

22. System according to one of the preceding claims, **characterized in that** it comprises, in the measurement arm, means of compensating for the effects of the focal chromatism of the eye.

23. System according to any one of the preceding claims, **characterized in that** it comprises, in the reference arm, means of compensating for the dispersion of the path differences.

24. *In vivo* tomography method with high axial and lateral resolution of the human retina, comprising:
- a full-field tomography by interference with low coherence length (OCT) with a Z scanning, using an input light source (S),
- a production of an image of the retina by means of detection (CCD), from an interferometric measurement according to the OCT principle,
- a correction of the wavefronts originating from the eye and reaching the eye, by adaptive optical means (MD, SLD, SH), arranged between the interferometer and the eye, comprising an analysis of the wave surface on the retina, and
- an adjustment of the focussing of the wave surface analysis,
**characterized in that** the focussing adjustment is carried out so as to conjugate the input light source (S) and the means of detection (CCD) with a point of predetermined depth in the retina, in synchronism with the Z scanning of the OCT tomography.

25. Method according to claim 24, **characterized in that** the interferometric measurement comprises a measurement of the contrast of the fringes without modulation by the method termed Wollaston.

26. Method according to one of claims 24 or 25, **characterized in that** it further comprises a compensation for the effects of birefringence of the cornea.

27. Method according to claim 26, **characterized in that** it further comprises a linear polarization (CPA) of the reference source (SLD) and a switching of the polarization between the outward and return paths in the arms.

28. Method according to one of claims 24 to 27, **characterized in that** it further comprises a filtering (DCM) of the corneal reflection.

29. Method according to one of claims 24 to 28, **characterized in that** it further comprises a tuning of the adjustment to a given depth, by controlling the adaptive optical means (MD) in reaction to an overall defocusing of the assembly constituted by the reference source (SLD) and the wave surface analyser means (SH).

30. Method according to one of claims 24 to 29, **characterized in that** it further comprises an adjustment of the focussing of the wave surface analyser means (SH).

31. Method according to one of claims 24 to 30, **characterized in that** it further comprises a freezing of the shape of the adaptive optical means (MD) for the duration of an exposure.

32. Method according to one of claims 24 to 31, **characterized in that** it comprises, in the measurement arm, a compensation for the effects of the focal chromatism of the eye.

33. Method according to one of claims 24 to 32, **characterized in that** it comprises, in the reference arm, a compensation for the dispersion of the path differences.

34. Method according to one of claims 24 to 33, **characterized in that** it comprises a command to the wavefront analyser (SH) obliging it to work in defocused mode.

## Patentansprüche

1. System zur In-vivo-Tomographie der menschlichen Netzhaut mit hoher axialer und lateraler Auflösung, das enthält:
- ein Michelson-Interferometer, das einen Vollfeld-Tomographie-Aufbau durch Interferenz mit geringer Kohärenzlänge (OCT) mit einer Z-Achse-Abtastung herstellt,
- eine Eingangslichtquelle (S), die in einem Eingangsarm des Interferometers angeordnet ist,
- adaptive Optikeinrichtungen, vorgesehen, um vom Auge kommende und zum Auge gehende Wellenfronten zu korrigieren, die eine Bezugsquelle (SLD), einen verformbaren Spiegel (MD) und Einrichtungen zur Wellenoberflächenanalyse (SH) enthalten,
- Erfassungseinrichtungen (CCD), die in einem Bildgebungsarm des Interferometers angeordnet sind und vorgesehen sind, um ein Bild ausgehend von einer Interferenzmessung gemäß dem Prinzip des OCT zu erzeugen, und
- Einrichtungen (LA2, LA3, LA4), um die Fokussierung der Einrichtungen zur Wellenoberflächenanalyse (SH) zu regeln, **dadurch gekennzeichnet, dass** die Einrichtungen zur Regelung der Fokussierung eingerichtet sind, um den verformbaren Spiegel zu zwingen, eine zusätzliche Krümmung anzunehmen, um die Eingangslichtquelle (S) und die Erfassungseinrichtungen (CCD) mit einem Punkt vorbestimmter Tiefe in der Netzhaut zu konjugieren, wobei die Regeleinrichtungen synchron mit der Z-Achse-Abtastung des Tomographieaufbaus OCT gesteuert werden.

2. System nach Anspruch 1, **dadurch gekennzeichnet, dass** die adaptiven Optikeinrichtungen (MD, SLD, SH) zwischen dem Michelson-Interferometer und dem zu untersuchenden Auge (OEX) angeordnet sind.

3. System nach einem der Ansprüche 1 oder 2, **dadurch gekennzeichnet, dass** es außerdem Einrichtungen enthält, um die adaptiven Optikeinrichtungen (MD) auf der Basis von Wellenfrontmessungen zu steuern, die hinter den adaptiven Optikeinrichtungen an einem punktförmigen Bild der Bezugsquelle (SLD) durchgeführt werden, das auf der Netzhaut des Auges hergestellt wird.

4. System nach Anspruch 3, **dadurch gekennzeichnet, dass** es außerdem Einrichtungen enthält, um einen vom Messstrahl unabhängigen, zusätzlichen Lichtstrahl einzuführen, der auf die Netzhaut fokussiert ist.

5. System nach einem der vorhergehenden Ansprüche, **dadurch gekennzeichnet, dass** die Einrichtungen zur Wellenoberflächenanalyse (SH) einen Analysator vom Typ Shack-Hartmann enthalten.

6. System nach einem der vorhergehenden Ansprüche, **dadurch gekennzeichnet, dass** es außerdem Einrichtungen (CBC) zur Kompensation der Doppelbrechungswirkungen der Hornhaut enthält, die vor dem Auge (OEX) angeordnet sind.

7. System nach einem der vorhergehenden Ansprüche, **dadurch gekennzeichnet, dass** die zwei Arme des Interferometers von geradlinig polarisiertem Licht durchlaufen werden.

8. System nach Anspruch 7, **dadurch gekennzeichnet, dass** es außerdem einen polarisierenden Würfel (CPR) enthält, um in jedem Arm zwei zueinander lotrechte Polarisierungen zu erhalten.

9. System nach Anspruch 8, **dadurch gekennzeichnet, dass** die zwei Arme des Interferometers Einrichtungen aufweisen, um die Polarisation zwischen dem Hin- und Rückweg um 90 Grad kippen zu lassen.

10. System nach Anspruch 9, **dadurch gekennzeichnet, dass** die Einrichtungen zum Kippen der Polarisation ein Viertelwellenlängenplättchen (QOR, QOM) enthalten.

11. System nach einem der Ansprüche 7 bis 10, **dadurch gekennzeichnet, dass** das Interferometer mit linear polarisiertem Licht (S, P) beleuchtet wird.

12. System nach einem der Ansprüche 7 bis 11, **dadurch gekennzeichnet, dass** es außerdem Einrichtungen enthält, um die Ausrichtung der geradlinigen Eingangspolarisation (P) so anzupassen, dass eine vorbestimmte Verteilung der in die zwei Arme des Interferometers eingespeisten Ströme erhalten wird.

13. System nach den Ansprüchen 6 und 10, **dadurch gekennzeichnet, dass** das Viertelwellenlängenplättchen (QOM) dem Auge am nächsten angeordnet ist, vor den Einrichtungen zur Kompensation der Doppelbrechung.

14. System nach einem der vorhergehenden Ansprüche, **dadurch gekennzeichnet, dass** es außerdem Einrichtungen enthält, um die Hornhautreflektion zu filtern.

15. System nach Anspruch 14, **dadurch gekennzeichnet, dass** die Einrichtungen zum Filtern der Hornhautreflektion eine Feldblende (DCM) enthalten, die angeordnet ist, um den größten Teil des von der Hornhaut reflektierten Stroms zu stoppen.

16. System nach einem der vorhergehenden Ansprüche, **dadurch gekennzeichnet, dass** es außerdem Einrichtungen enthält, um die Justierung auf eine gegebene Tiefe durch Reaktion der adaptiven Optikeinrichtungen (MD) auf eine globale Defokussierung der von der Bezugsquelle (SLD) und den Analyseeinrichtungen (SH) gebildeten Einheit anzupassen.

17. System nach einem der vorhergehenden Ansprüche, **dadurch gekennzeichnet, dass** es außerdem ein aktives Sehzeichen (MAM) enthält.

18. System nach einem der vorhergehenden Ansprüche, **dadurch gekennzeichnet, dass** es außerdem Einrichtungen enthält, um die Form der adaptiven Optikeinrichtungen (MD) während der Dauer einer Belichtung einzufrieren.

19. System nach einem der vorhergehenden Ansprüche, **dadurch gekennzeichnet, dass** die Bezugsquelle (SLD) vor den adaptiven Optikeinrichtungen (MD) angeordnet ist.

20. System nach einem der Ansprüche 1 bis 18, **dadurch gekennzeichnet, dass** die Bezugsquelle (SLD) in den optischen Weg zwischen den adaptiven Optikeinrichtungen (MD) und dem zu untersuchenden Auge (OEX) eingefügt wird.

21. System nach einem der vorhergehenden Ansprüche, **dadurch gekennzeichnet, dass** es Einrichtungen (IRIS) zur Verfolgung der Bewegung des zu untersuchenden Auges enthält, die mit den Regel- oder Erfassungseinrichtungen zusammenarbeiten.

22. System nach einem der vorhergehenden Ansprüche, **dadurch gekennzeichnet, dass** es im Messarm Einrichtungen zur Kompensation der Wirkungen des fokalen Chromatismus des Auges enthält.

23. System nach einem der vorhergehenden Ansprüche, **dadurch gekennzeichnet, dass** es im Bezugsarm Einrichtungen zum Kompensieren der Dispersion der Wegdifferenzen enthält.

24. Verfahren zur In-vivo-Tomographie der menschlichen Netzhaut mit hoher axialer und lateraler Auflösung, das enthält:
- eine Vollfeld-Tomographie durch Interferenz mit geringer Kohärenzlänge (OCT) mit einer Z-Achse-Abtastung, die eine Eingangslichtquelle (S) verwendet,
- eine Erzeugung eines Bilds der Netzhaut durch Erfassungseinrichtungen (CCD) ausgehend von einer Interferenzmessung gemäß dem Prinzip des OCT,
- eine Korrektur der vom Auge kommenden und zum Auge gehenden Wellenfronten durch zwischen dem Interferometer und dem Auge angeordnete adaptive Optikeinrichtungen (MD, SLD, SH), die eine Wellenoberflächenanalyse auf der Netzhaut enthalten, und
- eine Regelung der Fokussierung der Wellenoberflächenanalyse,
**dadurch gekennzeichnet, dass** die Fokussierregelung so durchgeführt wird, dass die Eingangslichtquelle (S) und die Erfassungseinrichtungen (CCD) mit einem Punkt vorbestimmter Tiefe in der Netzhaut synchron mit der Z-Achse-Abtastung der OCT-Tomographie konjugiert werden.

25. Verfahren nach Anspruch 24, **dadurch gekennzeichnet, dass** die Interferenzmessung eine Messung des Kontrasts der Streifen ohne Modulation durch die so genannte Wollaston'sche Methode enthält.

26. Verfahren nach einem der Ansprüche 24 oder 25, **dadurch gekennzeichnet, dass** es außerdem eine Kompensation der Doppelbrechungswirkungen der Hornhaut enthält.

27. Verfahren nach Anspruch 26, **dadurch gekennzeichnet, dass** es außerdem eine lineare Polarisation (CPA) der Bezugsquelle (SLD) und ein Kippen der Polarisation zwischen Hin- und Rückweg in den Armen enthält.

28. Verfahren nach einem der Ansprüche 24 bis 27, **dadurch gekennzeichnet, dass** es außerdem eine Filterung (DCM) der Hornhautreflektion enthält.

29. Verfahren nach einem der Ansprüche 24 bis 28, **dadurch gekennzeichnet, dass** es außerdem eine Anpassung der Justierung auf eine gegebene Tiefe enthält, indem die adaptive Optik (MD) als Reaktion auf eine globale Defokussierung der aus der Bezugsquelle (SLD) und den Einrichtungen zur Wellenoberflächenanalyse (SH) bestehenden Einheit gesteuert wird.

30. Verfahren nach einem der Ansprüche 24 bis 29, **dadurch gekennzeichnet, dass** es außerdem eine Regelung der Fokussierung der Einrichtungen zur Wellenoberflächenanalyse (SH) enthält.

31. Verfahren nach einem der Ansprüche 24 bis 30, **dadurch gekennzeichnet, dass** es außerdem ein Einfrieren der Form der adaptiven Optikeinrichtungen (MD) während der Dauer einer Belichtung enthält.

32. Verfahren nach einem der Ansprüche 24 bis 31, **dadurch gekennzeichnet, dass** es im Messarm eine Kompensation der Wirkungen des fokalen Chromatismus des Auges enthält.

33. Verfahren nach einem der Ansprüche 24 bis 32, **dadurch gekennzeichnet, dass** es im Bezugsarm Einrichtungen zum Kompensieren der Dispersion der Wegdifferenzen enthält.

34. Verfahren nach einem der Ansprüche 24 bis 33, **dadurch gekennzeichnet, dass** es eine Steuerung des Wellenfront-Analysators (SH) enthält, die ihn zwingt, defokussiert zu arbeiten.
